(19) Europäisches Patentamt
European Patent Office
Office européen des brevets

(11) Publication number: 0 000 285
B1

(12) EUROPEAN PATENT SPECIFICATION

(45) Date of publication of patent specification: 17.02.82

(21) Application number: 78300103.5

(22) Date of filing: 29.06.78

(51) Int. Cl.³: C 07 D 501/36, C 07 D 249/12, A 61 K 31/545

# (54) 7-Acylamino-3-((3-(carboxymethyl) thio-1 H-1,2,4-triazol-5-yl) thiomethyl)-3-cephem-4-carboxylic acid derivatives, processes for their preparation and compositions containing them.

(30) Priority: 30.06.77 US 811642

(43) Date of publication of application: 10.01.79 Bulletin 79/1

(45) Publication of the grant of the European patent: 17.02.82 Bulletin 82/7

(84) Designated Contracting States: BE CH DE FR GB LU NL SE

(56) References cited:
DE - A - 2 016 082
DE - A - 2 655 717
FR - A - 2 266 507
JP - A - 72 05550

CHEMICAL ABSTRACTS 85 (1976) 21397e.

(73) Proprietor: SMITHKLINE CORPORATION
1500 Spring Garden Street P.O. Box 7929
Philadelphia Pennsylvania 19101 (US)

(72) Inventor: Berges, David Alan
18 Buckwalter Road
Phoenixville Pennsylvania 19460 (US)

(74) Representative: Hargreaves, Gerald Henry, Dr. et al,
P.O.Box 39
Welwyn Garden City Hertfordshire AL7 1EY (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 000 285 B1

# 7-Acylamino-3-((3-(carboxymethyl) thio-1H-1,2,4-triazol-5-yl) thiomethyl)-3-cephem-4-carboxylic acid derivatives, processes for their preparation and compositions containing them

This invention relates to cephalosporin derivatives having antibacterial activity, to compositions containing them, and to a process for their preparation.

According to the present invention there is provided compounds of the formula (I):—

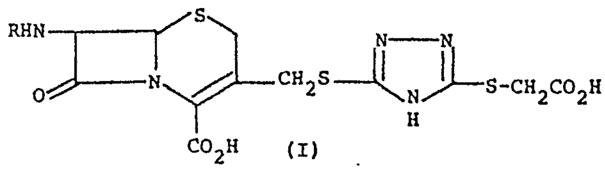

(I)

in which R is

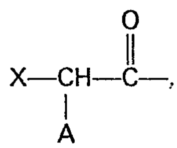 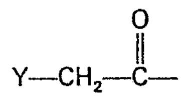 and 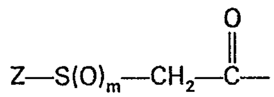

where

X is thienyl, furyl, phenyl, or phenyl monosubstituted by hydroxy, hydroxymethyl, formamido or ureido;

A is $NH_2$, OH, COOH, $SO_3H$, formyloxy or, when the $\alpha$-C-hydrogen is absent, methoxyimino;

Y is cyano, sydnonyl, pyridonyl, thienyl, *o*-aminomethylphenyl, phenyl or tetrazolyl;

Z is methyl, trifluoromethyl, trifluoroethyl, pyridyl or cyanomethyl;

m is zero to two;

and salts, esters and non-toxic amide derivatives thereof.

The cephem-4-carboxylic acid group, the carboxylic acid group of the —$SCH_2CO_2H$ moiety, the group A, when it is —$CO_2H$ or —OH can form esters. Accordingly herein an ester of a compound of formula (I) means an ester of one or more of these groups.

The carboxylic acid groups referred to above and the group A when A is —$SO_3H$ group can also form salts and the group A when it is —$NH_2$ can form acid addition salts. Accordingly herein a salt of a compound of formula (I) means a salt of the carboxyl or —$SO_3H$ group or an acid addition salt as the context requires.

When R is a 7-phenylglycylamino group, the amino group can form pharmaceutically acceptable amide derivatives. Accordingly, herein an amide derivative of a compound of formula (I) means a pharmaceutically acceptable non-toxic amide derivative of the compounds of formula (I) where R is 7-phenylglycylamino group.

Examples of salts of compounds of formula (I) include alkali metal salts for example sodium and potassium salts and salts with organic amines, in particular procaine and dibenzylethylenediamine salts.

Examples of esters of the compounds of formula (I) include simple alkyl and aryl esters and those which are easily cleaved in the body to the parent acid. Examples of these latter esters are indanyl, pivaloyloxymethyl, acetoxymethyl, propionoxymethyl, glyceryloxymethyl, phenylglycyloxymethyl and thienylglycyloxymethyl esters.

Examples of amide derivatives of the compounds of formula (I) are furyl-, pyranyl-, oxolanyl- and oxiranyl- carbonyl amides (i.e. Belgian Patent No. 835,295).

Compounds of formula (I) may also exist in the form of solvates for example hydrates, glycolates and alcoholates. Such forms are within the scope of the invention.

The acyl group R is preferably a group known to be of utility as a substituent on the 7-amino group in the structures of known or prior art cephalosporins or on the 6-amino group in the structures of known or prior art penicillins.

Representative 7-acylamino substituents of the compounds of formula (I) are listed below:—

$\alpha$-hydroxyphenylacetamido
$\alpha$-aminophenylacetamido
$\alpha$-amino-4-hydroxyphenylacetamido
trifluoromethylthioacetamido
2,2,2-trifluoroethylsulfinylacetamido
2,2,2-trifluoroethylthioacetamido
cyanoacetamido
$\alpha$-carboxythienylacetamido
$\alpha$-carboxyphenylacetamido

$\alpha$-sulfophenylacetamido
methylsulfonylacetamido
cyanomethylthioacetamido
3-sydnoneacetamido
1-tetrazolylacetamido
2-thienylacetamido
$\alpha$(Z)-(methoxyimino)-2-furanacetamido
4-pyridylthioacetamido
*o*-aminomethylphenylacetamido

Some compounds of formula (I) exist as optical isomers. For example where R is mandelamido or phenylglycylamido. The D-form of these subgeneric groups are preferred.

Compounds of formula (I) can be prepared by a process which comprises reacting a compound of formula (II):—

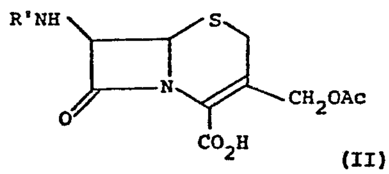

(II)

where R′ is hydrogen or a group R as defined with reference to formula (I) provided that any free amino, carboxy, or sulpho groups are optionally protected and Ac is acetyl, with a compound of formula (III):—

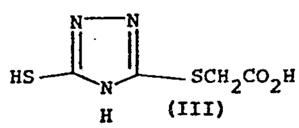

(III)

or an alkali metal salt thereof and where R′ is hydrogen acylating the product so obtained with an acylating agent or active derivative of formula R—OH where R is as defined with reference to formula (I) provided that any free amino, carboxy or sulpho groups are optionally protected, thereafter removing any protecting groups present, and optionally converting the compounds of formula (I) into a salt, ester or non-toxic amide derivative.

Compounds of formula (III) are disclosed and claimed in our co-pending European Patent Application No. 0015631.

N-acylation procedures may be found in Cephalosporins and Penicillins, Flynn Academic Press, 1972, U.S. Patent Nos. 2,721,196 and 3,953,424; Belgian Patent No. 832,725 and German Patent Nos. 2,127,285 and 2,406,165.

Esterification of the carboxylic acid groups of compounds of formula (I) can be carried out by known methods.

Salts of compounds of formula (I) can be prepared by known methods, for example sodium and potassium salts can be prepared by reacting the acid with sodium or potassium 2-ethyl hexanoate.

Pharmaceutically acceptable non-toxic amide derivatives can be prepared by analogy with known methods for example as described in Belgian Patent No. 835,295.

Protecting groups suitable for use in the above process are known (see "Protective Groups in Organic Chemistry" J.F.N. McOmie, Plenum Press, 1973, Chapters 2 and 3) for amino, carboxy, sulpho or hydroxy protecting groups.

Examples of such groups are *t*-butyl, for COOH or *t*-butoxy carbonyl for $NH_2$.

The protecting groups can be removed by known methods for example *t*-butyl and *t*-butoxycarbonyl, can be removed by trifluoracetic acid.

The compounds of formula (I) have antibacterial activity against both Gram positive and Gram negative bacteria, and minimum inhibitory concentrations (MIC's) in *vitro* of from 0.2 to greater than 200 $\mu$g/ml have been observed. Test results for 7-D-mandelamino-3-[[3-(carboxymethyl)thio-1*H*-1,2,4-triazol-5-yl]thiomethyl]-3-cephem-4-carboxylic acid, disodium salt, hydrate (A) are:

| | A | Cephalothin | Cefazolin |
|---|---|---|---|
| *S. aureus* HH 127 | 1.6 | 0.4 | 0.4 |
| *S. aureus* SK 23390 | 0.8 | 0.2 | 0.4 |
| *S. aureus villaluz* SK 70390 | 200 | 50 | 200 |
| *Strep. faecalis* HH 34358 | 100 | 12.5 | 6.3 |
| *E. coli* SK 12140 | 0.8 | 3.1 | 1.6 |
| *E. coli* HH 33779 | 0.8 | 6.3 | 1.6 |
| *Kleb. pneumo.* SK 4200 | 0.4 | 3.1 | 1.6 |
| *Kleb. pneumo.* SK 1200 | 0.2 | 1.6 | 0.8 |
| *Salmonella ATCC* 12176 | 0.8 | 1.6 | 0.8 |
| *Pseudo. aeru.* HH 63 | 200 | 200 | 200 |
| *Serratia marc.* ATCC 13880 | 12.5 | 200 | 200 |
| *Proteus morgani* 179 | 1.6 | 200 | 200 |
| *Entero. aeroq.* ATCC 13048 | 1.6 | 12.5 | 1.6 |
| *Entero cloacae* HH 31254 | 0.8 | 6.3 | 0.8 |

Compound A showed an $ED_{50}$ in mice of 1.56 against *E.coli* as well as 1.02 mg/kg against *Kleb. pneumo* (s.c.).

These results are superior to those for a related compound, 7-D-mandelamino-3-(2-carboxymethylthio-1,3,4-thiadiazol-5-yl-thiomethyl)-3-cephem-4-carboxylic acid, sodium salt hydrate which showed an $ED_{50}$ agains *E.coli* of 6.4 and as well as 4.4 mg/kg. against *Kleb. pneumo.*

Pharmaceutical compositions having antibacterial activity which comprise a pharmaceutical carrier containing a compound of formula (I), pharmaceutically acceptable salt, non-toxic amide derivative or ester thereof which is easily cleaved within the body to the parent acid, and their use as antibacterial agents by administering them in a composition in a non-toxic amount sufficient to combat such infections are also within this invention. The administration may be orally or by parenteral injection such as subcutaneously, intramuscularly, or intravenously. The injection of suitably prepared sterile solutions or suspensions containing an effective, non-toxic amount of a cephalosporin compound of the invention is the preferred route of administration.

The compositions of this invention are preferably formulated and administered in the same manner as other prior art cephalosporins such as cephazolin or cephalothin. The dosage regimen preferably comprises administration, preferably by injection, of an active but non-toxic quantity of a compound of formula (I) from about 250 mg. to 600 mg. with the total daily dosage regimen being from about 750 mg. to 6 g. The precise dosages are dependant upon the age and weight of the subject and on the susceptibility of the infection being treated to each individual. These can be determined by those skilled in the art based on the data disclosed herein compared with that available to the art attained with the known cephalosporins outlined herebefore. The following Examples illustrate the invention. All temperatures are in °C.

EXAMPLE 1

To a suspension of 5.48 g. (40 mmol) of 1,2,4-triazolidine-3,5-dithione, monohydrazine salt in 80 ml. of tetrahydrofuran and 80 ml. of dimethylformamide was added a solution of 6.7 g. (40 mmol) of ethyl bromoacetate in 20 ml. of tetrahydrofuran. The mixture was stirred at room temperature for 1-1/2 hours, and then at 50°C. for 1/2 hour. The solution was filtered and the filtrate was concentrated to 80 ml., diluted with 250 ml. of water and extracted with diethyl ether. The extract was dried ($MgSO_4$) and evaporated to dryness to give a residue which was crystallized from methanol and water to give 5.76 g. (65% yield) of ethyl [(4,5-dihydro-5-thioxo-1*H*-1,2,4-triazol-3-yl)thio]acetate. mp 134—6°C.

A solution of 2.19 g. (10 mmol) of ethyl [4,5-dihydro-5-thioxo-1*H*-1,2,4-triazol-3-yl)thio]acetate in 50 ml. of 5% sodium hydroxide was heated at reflux for 1 hour. After thorough cooling, the mixture was filtered, and the filtrate was washed with ethyl acetate. The aqueous layer was separated, acidified

to pH 1 and extracted with ethyl acetate. The extract was evaporated *in vacuo* to dryness. The residue was crystallized from chloroform to give 1.43 g. (75% yield) of [(4,5-dihydro-5-thioxo-1H-1,2,4-triazol-3-yl)thio]acetic acid.

To a solution of 1.16 g. (13.8 mmol) of sodium bicarbonate in 30 ml. of water was added 1.32 g. (6.9 mmol) of [4,5-dihydro-5-thioxo-1*H*-1,2,4-triazol-3-yl)thio]acetic acid. After $CO_2$ gas evolution had ceased, 2.12 g. (5 mmol) of 7-mandelamido-cephalosporanic acid sodium salt was added to the solution. The mixture was heated at 80°C. for 3.5 hours. After cooling, the solution was filtered. The filtrate was applied to an XAD—7 (200 ml.) resin column, eluting with water. The fractions containing product by thin layer chromatography were pooled and evaporated to dryness. The residue was triturated with absolute ethanol and filtered. The filtrate was diluted with isopropanol, and the solid formed was filtered, air-dried, dissolved in de-ionized water and lyophilized to give 1.02 g. (15.6% yield) of 7 - D - mandelamido - 3 - [[3 - (carboxymethyl)thio - 1*H* - 1,2,4 - triazol - 5 - yl]thiomethyl] - 3 - cephem - 4 - carboxylic acid, disodium salt hydrate, mp 220—230°C. dec. Anal. calculated for $C_{20}H_{17}N_5O_7S_3Na_2.4H_2O$ = C, 36.75; H, 3.85; N, 10.71. Found: C, 36.47; H, 3.53; N, 10.11. IR—(NUJOL) = 5.65 $\mu$.

EXAMPLE 2

An aqueous solution (100 ml.) of 4.27 g (0.0096 mol) of 7-[$\alpha$(Z)-(methoxyimino)-2-furan-acetamido]cephalosporanic acid sodium salt, 1.78 g. (0.0212 mol) of sodium bicarbonate and 2.02 g. (0.0106 mol) of [(4,5-dihydro-5-thioxo-1*H*-1,2,4-triazol-3-yl)thio]acetic acid is heated at 65°C. for 6 hours during which time the pH is maintained at 7.6—7.8 with dilute sodium bicarbonate. After cooling, the reaction mixture is purified on an XAD—7 column as described in Example 1 to give a lyophilized product, 7-[$\alpha$(Z)-(methoxyimino)-2-furanacetamido]-3-[[3-(carboxymethyl) thio 1*H*-1,2,4-triazol-5-yl]thiomethyl]-3-cephem-4-carboxylic acid, disodium salt.

EXAMPLE 3

A mixture of 5.22 g. (10.0 mmol) of 7-(D-*t*-butoxycarbonylamino-4-hydroxyphenylacetamido)-cephalosporanic acid and an excess (15.0 mmol) of [(4,5-dihydro-5-thioxo-1*H*-1,2,4-triazol-3-yl)thio]-acetic acid in 75 ml. of pH 6.4 phosphate buffer solution is treated with sufficient sodium bicarbonate to give a pH of 6.4. The mixture is heated at 70° for 3 hours, cooled, acidified with dilute hydrochloric acid to pH 2 and extracted with ethyl acetate. Removal of the ethyl acetate *in vacuo* gives the *t*-boc derivative of the desired compound. This derivative is stirred at 25°C. with 25 ml. of trifluoroacetic acid and 25 ml. of 1,3-dimethoxybenzene for 2 hours. The mixture is evaporated to dryness *in vacuo*, ethyl acetate is added to the residue and the precipitated salt is collected. This is dissolved in water and treated with Amberlite IR—45 weakly basic ion-exchange resin. The solution is lyophilized to give 7-(D-$\alpha$ - amino - 4 - hydroxyphenylacetamido) - 3 - [[3 - carboxymethyl)thio - 1*H* - 1,2,4 - triazol - 5 - yl]thiomethyl] - 3 - cephem - 4 - carboxylic acid.

EXAMPLE 4

A mixture of an excess (12.2 mmol) of [(4,5-dihydro-5-thioxo-1*H*-1,2,4-triazol-3-yl)thio]acetic acid, 32.5 mmol of sodium bicarbonate and 8.1 mmol of 7-trifluoromethylthioacetamidocephalosporanic acid in 50 ml. of water is stirred at 70° for 5 hours. The reaction mixture is cooled and applied to an XAD—2 column and eluted with water and then methanol. The product containing effluent is evaporated to dryness to give a residue which is dissolved in a small amount of water and lyophilized to give 7-trifluoromethylthioacetamido-3-[[3-(carboxymethyl)thio-1*H*-1,2,4-triazol-5-yl]thiomethyl]-3-cephem-4-carboxylic acid disodium salt. Substituting 7-(2-thienylacetamido)-cephalosporanic acid gives 7-(2-thienylacetamido)-3-[[3-(carboxymethyl)thio-1*H*-1,2,4-triazol-5-yl]thiomethyl]-3-cephem-4-carboxylic acid sodium salt.

Stoichiometric quantities of cephalosporanic acids having the individual 7-acylamino substituent listed hereabove may be substituted in Examples 1—3 with variations which will be obvious to those skilled in this art.

EXAMPLE 5

An injectable pharmaceutical composition is formed by adding sterile saline solution (2 ml.) to 500 mg. of the product of Example 1. This material is injected parenterally four times daily into a human patient infected with susceptible bacteria. Other compounds of this invention may be similarly used.

The abbreviation "dec" used herein means decomposition.

## Claims

1. A compound of formula (I):—

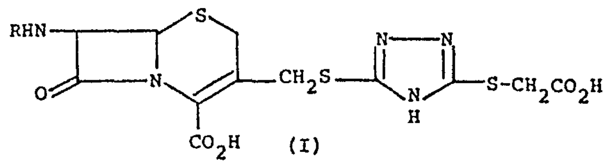

(I)

in which R is

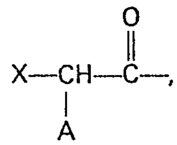, 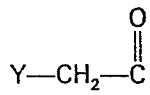 and 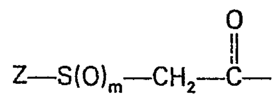

where

X is thienyl, furyl, phenyl or phenyl monosubstituted with hydroxy, hydroxymethyl, formamido or ureido;

A is $NH_2$, OH, COOH, $SO_3H$, formyloxy or, when the $\alpha$-carbon hydrogen is absent, methoxyimino;

Y is cyano, sydnonyl, pyridonyl, thienyl, o-aminomethylphenyl, phenyl or tetrazolyl;

Z is methyl, trifluoromethyl, trifluoroethyl, pyridyl or cyanomethyl;

m is zero to two;

and salts, esters and non-toxic amide derivatives thereof.

2. The D-form of an optically active compound as claimed in claim 1.

3. A compound of formula (I) as claimed in claim 1 and esters thereof which are easily cleaved in the body to the parent acid and pharmaceutically acceptable salts thereof, and non-toxic amide derivatives thereof.

4. 7 - D - mandelamido - 3 - [[3 - (carboxymethyl)thio - 1*H* - 1,2,4 - triazol - 5 - yl]thiomethyl] - 3 - cephem - 4 - carboxylic acid.

5. 7 - [$\alpha$(Z) - (methoxyimino) - 2 - furanacetamido] - 3 - [[3 - (carboxymethyl)thio - 1*H* - 1,2,4 - triazol - 5 - yl]thiomethyl] - 3 - cephem - 4 - carboxylic acid disodium salt.

6. 7 - trifluoromethyl(thioacetamido - 3 - [[3 - (carboxymethyl)thio -1*H* - 1,2,4 - triazol - 5 - yl]-thiomethyl] - 3 - cephem - 4 - carboxylic acid disodium salt.

7. A process for preparing a compound as claimed in claim 1 which comprises reacting a compound of formula (II):—

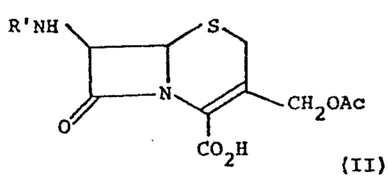

(II)

where R' is hydrogen or a group R as defined with reference to formula (I) provided that any free amino carboxy or sulpho groups are optionally protected, and Ac is acetyl, with a compound of formula (III):—

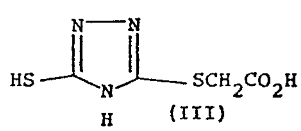

(III)

or an alkali metal salt thereof and where R' is hydrogen acylating the product with an acylating agent or active derivative of formula ROH where R is as defined with reference to formula (I) provided that any free amino, carboxy or sulpho groups are optionally protected, thereafter removing any protecting groups present and optionally converting the compound of formula (I) into a salt, ester or non-toxic amide derivative.

8. A pharmaceutical composition comprising a compound as claimed in any one of claims 3 to 6 and a pharmaceutically acceptable carrier.

**Patentansprüche**

Deutsche Übersetzung nach Artikel 97 (5) EPÜ in Verbindung mit Regel 51 (4)

1. Eine Verbindung der Formel I

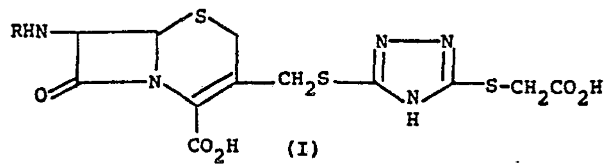

(I)

in der R die Gruppen

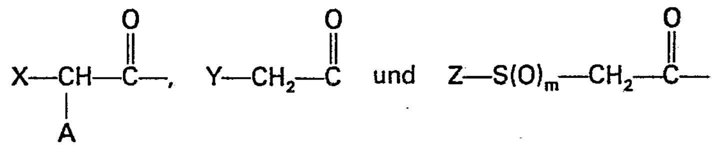

und

in denen X eine Thienyl- oder Furylgruppe oder eine gegebenenfalls mit einer Hydroxy-, Hydroxymethyl-, Formamido- oder Ureidogruppe einfach substituierte Phenylgruppe ist;

A $NH_2$, OH, COOH, $SO_3H$, eine Formyloxygruppe oder bei Abwesenheit von Wasserstoff ein $\alpha$-Kohlenstoffatom eine Methoxyiminogruppe ist;

Y eine Cyano-, Sydnonyl-, Pyridonyl-, Thienyl-, o-Aminomethylphenyl-, Phenyl- oder Tetrazolylgruppe ist;

Z eine Methyl-, Trifluoromethyl-, Trifluoräthyl-, Pyridyl- oder Cyanomethylgruppe ist;

m den Wert 0 bis 2 hat;

und ihre Salze, Ester und nicht toxischen Amidderivate.

2. Die D-Form einer optisch aktiven Verbindung nach Anspruch 1.

3. Eine Verbindung der Formel I nach Anspruch 1 und ihre Ester, die sich im Körper leicht in ihre Säure und pharmazeutisch verträglichen Salze spalten läßt und ihre nichttoxischen Amidderivate.

4. 7 - D - mandelamido - 3 - [[3 - (carboxymethyl) - thio - 1H - 1,2,4 - triazol - 5 - yl] - thiomethyl] - 3 - cephem - 4 - carbonsäure.

5. 7 - [$\alpha$(Z) - (methoxyimino) - 2 - furanacetamido] - 3 - [[3 - (carboxymethyl) - thio - 1H - 1,2,4 - triazol - 5 - yl]thiomethyl] - 3 - cephem - 4 - carbonsäure-dinatriumsalz.

6. 7 - Trifluormethyl - (thioacetamido - 3 - [[3 - (carboxymethyl) - thio - 1H - 1,2,4 - triazol - 5 - yl] - thiomethyl] - 3 - cephem - 4 - carbonsäure-dinatriumsalz.

7. Ein Verfahren zur Herstellung einer Verbindung nach Anspruch 1, gekennzeichnet durch Umsetzung einer Verbindung der Formel II

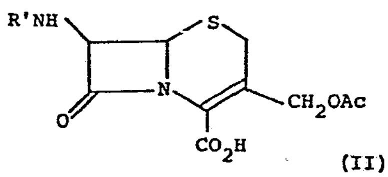

(II)

in der R′ ein Wasserstoffatom oder einen Rest R, wie er für die Formel I definiert ist, bedeutet, mit der Maßgabe, daß etwaige freie Amino-, Carboxy- oder Sulfogruppen gegebenenfalls geschützt sind, und Ac eine Acetylgruppe ist, mit einer Verbindung der Formel III

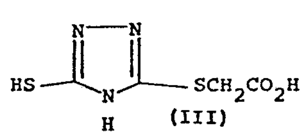

(III)

oder ihrem Alkalimetallsalz, wobei, falls R′ ein Wasserstoffatom ist, die Verbindung mit einem Acylierungsmittel oder einem aktiven Derivat der Formel ROH, in der R dieselbe Bedeutung wie in der Formel I hat, acyliert wird, mit der Maßgabe, daß etwaige freie Amino-, Carboxy- oder Sulfogruppen gegebenenfalls geschützt werden, anschließend etwaige Schutzgruppen entfernt werden und gegebenenfalls die Verbindung der Formel I in ein Salz, einen Ester oder ein nicht-toxisches Amidderivat überführt wird.

8. Eine pharmazeutische Zusammensetzung, enthaltend eine Verbindung nach einem der Ansprüche 3 bis 6 und einen pharmazeutisch verträglichen Träger.

**Revendications**

1. Composé de formule (I):

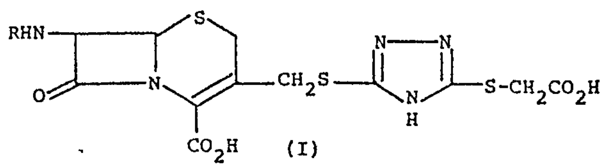

(I)

dans laquelle R représente

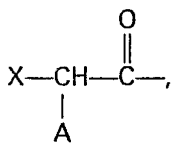, 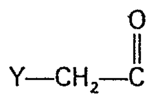 and 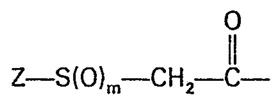

où

X est un groupe thiényle, furyle, phényle ou phényle monosubstitué par un groupe hydroxy, hydroxyméthyle, formamido ou uréido;

A est un groupe $NH_2$, OH, COOH, $SO_3H$, formyloxy ou, lorsque l'$\alpha$-carbone hydrogène est absent, méthoxyimino;

Y est un groupe cyano, sydnonyle, pyridonyle, thiényle, *o*-aminométhylphényle, phényle ou tétrazolyle;

Z est un groupe méthyle, trifluorométhyle, trifluoroéthyle, pyridyle ou cyanométhyle;

m varie de zéro à deux;

et ses sels, esters et dérivés amidés non-toxiques.

2. La forme D d'un composé optiquement actif tel que revendiqué dans la revendication 1.

3. Composé de formule (I) tel que revendiqué dans la revendication 1, ses esters qui sont aisément scindés en acide parent dans l'organisme, ses sels pharmaceutiquement acceptables et ses dérivés amidés non-toxiques.

4. Acide 7 - D - mandélamido - 3 - [[3 - (carboxyméthyl)thio - 1*H* - 1,2,4 - triazol - 5 - yl]thiométhyl] - 3 - cephème - 4 - carboxylique.

5. Sel disodique de l'acide 7 - [$\alpha$(Z) - méthoxyimino) - 2 - furanacétamido] - 3 - [[3 - (carboxyméthyl)thio - 1*H* - 1,2,4 - triazol - 5 - yl]thiométhyl] - 3 - cephème - 4 - carboxylique.

6. Sel disodique de l'acide 7 - trifluorométhyl(thioacétamido - 3 - [[3 - (carboxyméthyl)thio - 1*H*,1,2,4 - triazol - 5 - yl]thiométhyl] - 3 - cephème - 4 - carboxylique.

7. Procédé de préparation d'un composé tel que revendiqué dans la revendication 1, caractérisé en ce qu'on fait réagir un composé de formule (II):

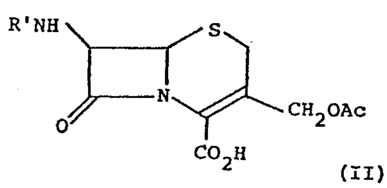

(II)

dans laquelle R' représente de l'hydrogène ou un groupe R tel que défini pour la formule (I) à condition que tout groupe amino, carboxy ou sulfo libre soit, si on le désire, protégé et Ac représente un groupe acétyle, avec un composé de formule (III):

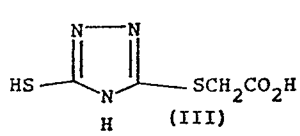

(III)

ou un de ses sels alcalins et, lorsque R' représente de l'hydrogène, on acyle le produit avec un agent d'acylation ou un dérivé actif de formule ROH dans laquelle R est tel que défini pour la formule (I), à condition que tout groupe amino, carboxy ou sulfo libre soit, si on le désire, protégé, on enlève ensuite tout groupe protecteur et, si on le désire, on transforme le composé de formule (I) en un sel, ester ou dérivé amidé non-toxique.

8. Composition pharmaceutique comprenant un composé tel que revendiqué dans l'une quelconque des revendications 3 à 6 avec un excipient pharmaceutiquement acceptable.